# EUROPEAN PATENT SPECIFICATION

(11) **EP 1 888 157 B1**
(45) Date of publication and mention of the grant of the patent: **31.07.2013**
(21) Application number: 06744116.2
(22) Date of filing: 06.06.2006
(51) Int. Cl.: A61M 16/04, A61M 16/08

(54) **MEDICO-SURGICAL APPARATUS**
MEDIZINISCH-CHIRURGISCHE VORRICHTUNG
APPAREIL MÉDICO-CHIRURGICAL

(30) Priority: 07.06.2005 GB 0511517
(43) Date of publication of application: 20.02.2008
(73) Proprietor: Smiths Group plc, London SW1E 5JL (GB)
(72) Inventor: HUDDLESTONE, Mark James, Hythe, Kent CT21 4PJ (GB); GRAHAM, Mark Andrew, Folkeston e, Kent CT19 4QG (GB); WILLIAMS, Lois Rebecca, St Ouen, Jersey JE3 2FF (GB)
(74) Representative: Flint, Jonathan McNeill
(86) International application number: PCT/GB2006/002062
(87) International publication number: WO 2006/131719

(56) References cited:
- WO-A-03/105938
- US-A- 2 845 928
- US-A1- 2001 047 804
- US-A1- 2003 154 980
- US-B1- 6 668 831

## Description

This invention relates to gas-treatment devices of the kind having a tubular housing, a port located substantially midway along the housing and adapted for connection to a patient breathing device such that the housing extends generally transversely of the port to provide two open end portions projecting either side of the port, and first and second gas-treatment elements located towards opposite ends of the respective end portions through which gas can flow to the patient.

The invention is more particularly concerned with devices such as heat and moisture exchangers (HMEs) or filters.

Where a patient breathes through a tube inserted in the trachea, such as a tracheostomy or endotracheal tube, gas flow to the bronchi is not warmed and moistened by passage through the nose. Unless the gas is warmed and moistened in some way it can cause damage and discomfort in the patient's throat. The gas can be conditioned by a humidifier in the ventilation circuit but, most conveniently, a heat and moisture exchange device (HME) is used. HMEs are small, lightweight devices including one or more exchange elements, such as of a paper or foam treated with a hygroscopic substance. When the patient exhales, gas passes through the exchange element and gives up a major part of its heat and moisture to the element. When the patient inhales, gas passes through the exchange element in the opposite direction and takes up a major part of the heat and moisture in the exchange element so that the gas inhaled by the patient is warmed and moistened. These HMEs are low cost and disposable after a single use so do not require cleaning or present any cross contamination risk. They can be connected in a breathing circuit or simply connected to the machine end of a tracheal tube and left open to atmosphere where the patient is breathing spontaneously.

HMEs are sold by Smiths Medical International Limited of Hythe, Kent, England under the Thermovent name (Thermovent is a registered trade mark of Smiths Medical International Limited), by Hudson RCI AB under the TrachVent name (TrachVent is a registered trade mark of Hudson RCI AB), by DAR, Medisize, Intersurgical and other manufacturers. Examples of HMEs are described in GB 2391816, WO 01/72365,
US 5505768, SE 516666, US 3881482, DE 20302580, DE 20114355U,
WO 97/01366, US 2002/0157667, US 6422235, EP 1208866, US 4971054, PCT/GB05/005065 and PCT/GB04/00503. The "Thermovent T" HME sold by Smiths Medical has a T-shape configuration with two HME elements mounted at opposite ends of a straight tubular housing extending transversely of the connection port by which the device is fitted onto a tracheotomy tube or the like. Another example of gas-treatment devices can be found in US6668831.

It is preferable for HMEs or the like to be as compact as possible in order to reduce the risk that they will catch on clothing and to help make them inconspicuous.

It is an object of the present invention to provide an alternative gas-treatment device.

According to the present invention there is provided a gas-treatment device of the above-specified kind, characterised in that the tubular housing is curved along its length such that the opposite end portions of the housing on opposite sides of the port curve towards the port and the patient.

The tubular housing is preferably elongated in section such that it is wider in a plane at right angles to its plane of curvature. The housing may have an oval section. The gas-treatment elements are preferably HME elements and each may include a spiral roll of hygroscopic treated paper. The device preferably has a suction access aperture located opposite the port.

An HME according to the present invention will now be described, by way of example, with reference to the accompanying drawings, in which:
- Figure 1: is a plan view of the HME;
- Figure 2: is a perspective end view of the HME from one side;
- Figure 3: is a perspective view of the HME in use on a tracheostomy tube with a suction catheter making access via a suction port; and
- Figures 4 to 6: are perspective views of three alternative HMEs.

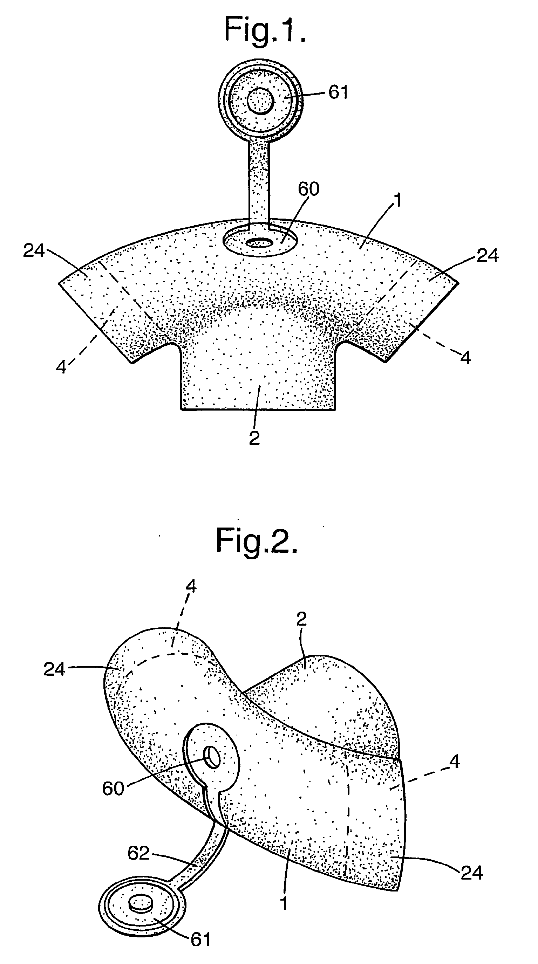

With reference first to Figures 1 to 3, the HME has an outer housing 1 with a coupling or port 2 by which it is connected to a tracheostomy tube 3. The housing 1 supports two heat and moisture exchange elements 4 at opposite ends by which gas supplied to the patient is warmed and moistened.

The housing 1 is tubular with a genreally T shape and is moulded from a rigid plastics material. The patient coupling or port 2 is hollow with a circular section and projects radially outwardly, laterally of the housing midway along its length. It has an internal tapered surface adapted to connect to a standard male tapered coupling (not shown) on the end of the tracheostomy tube 3. The coupling 2 opens to the interior 22 of the housing 1 in communication with the inner surface of the exchange elements 4.

The exchange elements 4 are mounted at opposite ends of the housing 1. The housing 1 differs from conventional HMEs in that it is curved along its length in an arc shaped to follow approximately the surface of the neck on either side of the tracheostomy. Opposite ends 24 of the housing 1, therefore, curve forwardly towards the patient port 2. The housing 1 also differs in that, instead of having a circular section, as in conventional HMEs, the housing 1 is elongated in a plane at right angles to its plane of curvature so that it has an oval shape. This reduces slightly the extent to which the HME projects outwardly, making it more compact. This, and the curved shape of the housing 1, reduces the risk that it will catch on clothing and helps make the HME appear less obtrusive. The curve to the housing 1 also means that the open, opposite ends 24 of the housing are directed slightly rearwardly, which makes them less prone to being occluded, such as by clothing. The exchange elements 4 are each conventional, being in the form of a disc comprising a spiral roll of corrugated paper treated with a hygroscopic salt to promote the retention of moisture. The exchange elements 4 extend transversely of the axis of the housing 1 at opposite ends 24 and are squashed slightly to the oval shape of the cross section of the housing.

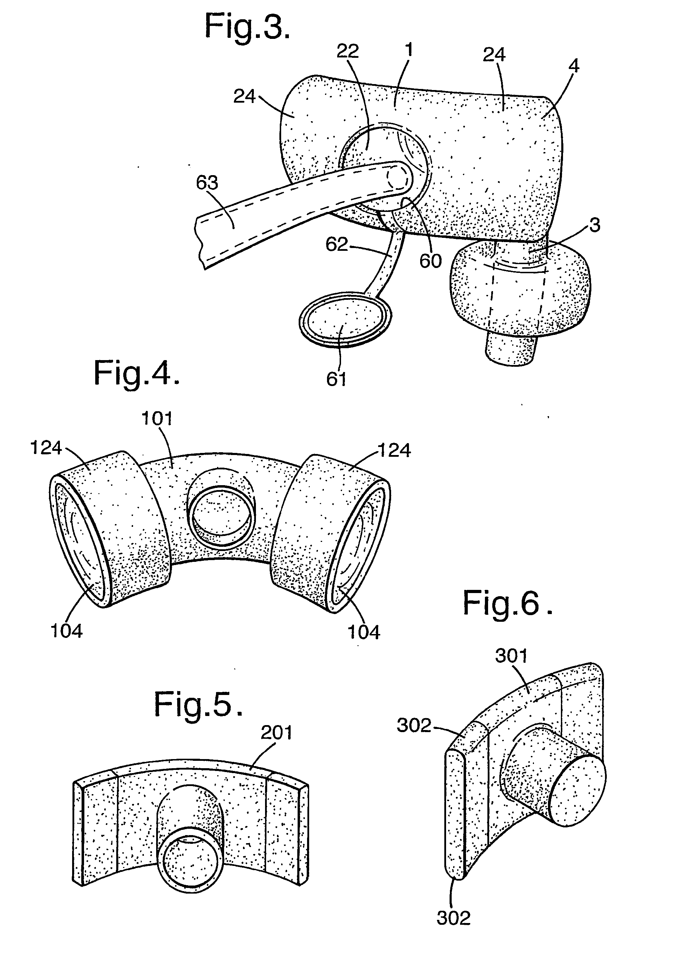

The HME also has a suction access aperture 60 located directly opposite the tracheal tube coupling 2. The aperture 60 is circular and is normally covered and closed by a cover or flap 61 formed integrally with the housing 1 and attached with it at one end by a web or living hinge 62. The hinge 62 is bendable to allow the flap 61 to be raised or lowered over the aperture 60. When the patient's tracheal tube 3 needs suctioning, the clinician lifts the flap 61 and inserts a suction tube 63 down the tracheal tube through the patient coupling 2, as shown in Figure 3. This avoids the need to remove the HME. The fit of the flap 61 in the aperture 60 could be arranged such that the flap can be blown outwardly by increased pressure created by the patient, such as when coughing. This would provide a pressure relief feature.

The HME may also include an oxygen supply port (not shown), of conventional kind by which additional oxygen can be supplied to the gas inhaled by the patient.

Figure 4 shows a slightly modified HME where opposite ends 124 of the housing 101 are enlarged in cross section to receive exchange elements 104.

The housing 1 need not have an oval section as shown in Figures 1 to 4. Instead, as shown in Figure 5, the housing 201 could be elongated with a rectangular section, or, as shown in Figure 6, the housing 301 could have a section that is rectangular with rounded ends 302.

The HME is not confined to use on tracheostomy tubes but could be used on other breathing devices such as endotracheal tubes or face masks.

It will be appreciated that the invention is not confined to HMEs but could be used with other devices such as where the gas-treatment elements are filters.

## Claims

1. A gas-treatment device having a tubular housing (1, 101, 201, 301), a port (2) located substantially midway along the housing and adapted for connection to a patient breathing device (3) such that the housing extends generally transversely of the port to provide two open end portions (4, 124) projecting either side of the port, first and second gas-treatment elements (4, 104) located towards opposite ends of the respective end portions through which gas can flow to the patient, **characterised in that** the tubular housing (1, 101, 201, 301) is curved along its length such that the opposite end portions (4, 124) of the housing on opposite sides of the port (2) curve towards the port (2) and the patient.

2. A gas-treatment device according to Claim 1, **characterised in that** the tubular housing (1, 101, 201, 301) is elongated in section such that it is wider in a plane at right angles to its plane of curvature.

3. A gas-treatment device according to Claim 2, **characterised in that** the housing (1, 101) has an oval section.

4. A gas-treatment device according to any one of the preceding claims, **characterised in that** the gas-treatment elements are HME elements (4, 104).

5. A gas-treatment device according to Claim 4, **characterised in that** each HME element includes a spiral roll of hygroscopic treated paper (4, 104).

6. A gas-treatment device according to any one of the preceding claims, **characterised in that** the device has a suction access aperture (60) located opposite the port (2).

## Revendications

1. Dispositif de traitement de gaz comportant un logement tubulaire (1, 101, 201, 301), un orifice (2) situé sensiblement à mi-chemin le long du logement et adapté pour la connexion à un dispositif respiratoire d'un patient (3) tel que le logement s'étend généralement transversalement à l'orifice pour fournir deux extrémités de portions ouvertes (4, 124) faisant saillies de chaque côté de l'orifice, des premier et second éléments de traitement de gaz (4, 104) situés vers les extrémités opposées des extrémités de portions respectives au travers desquelles le gaz s'écoule vers le patient, **caractérisé en ce que** le logement tubulaire (1, 101, 201, 301) est incurvé sur sa longueur tel que les extrémités opposées de portions (4, 124) du logement sur le côté opposé à l'orifice (2) s'incurvent vers l'orifice (2) et le patient.

2. Dispositif de traitement de gaz selon la revendication 1, **caractérisé en ce que** le logement tubulaire (1, 101, 201, 301) est de section allongée de telle sorte qu'il est plus large dans un plan perpendiculaire à son plan de courbure.

3. Dispositif de traitement de gaz selon la revendication 2, **caractérisé en ce que** le logement tubulaire (1, 101, 201, 301) comporte une section ovale.

4. Dispositif de traitement de gaz selon l'une quelconque des revendications précédentes, **caractérisé en ce que** les éléments du traitement de gaz sont des éléments HME (4, 104).

5. Dispositif de traitement de gaz selon la revendication 4, **caractérisé en ce que** chaque élément HME inclut un rouleau en spirale de papier traité hygroscopique (4, 104).

6. Dispositif de traitement de gaz selon l'une quelconque des revendications précédentes, **caractérisé en ce que** le dispositif comporte une ouverture d'accès à une aspiration (60) située à l'opposé de l'orifice (2).

## Patentansprüche

1. Gasbehandlungsvorrichtung mit einem rohrförmigen Gehäuse (1, 101, 201, 301), einem Anschluss (2), der im Wesentlichen mittig längs des Gehäuses angeordnet ist und derart zur Verbindung mit einer Patientenbeatmungsvorrichtung (3) angepasst ist, dass sich das Gehäuse im Wesentlichen quer zum Anschluss erstreckt, um zwei auf jeder Seite des Anschlusses überstehende offene Endabschnitte (4, 124) bereitzustellen, wobei erste und zweite Gasbehandlungselemente (4, 104) auf gegenüberliegenden Enden der jeweiligen Endabschnitte, durch welche Gas zu den Patienten fließen kann, angeordnet sind, **dadurch gekennzeichnet, dass** das rohrförmige Gehäuse (1, 101, 201, 301) längs seiner Länge derart gekrümmt ist, dass die gegenüberliegenden Endabschnitte (4, 124) des Gehäuses auf gegenüberliegenden Seiten des Anschlusses (2) zu dem Anschluss (2) und dem Patienten hin gekrümmt sind.

2. Gasbehandlungsvorrichtung nach Anspruch 1, **dadurch gekennzeichnet, dass** das rohförmige Gehäuse (1, 101, 201, 301) im Querschnitt länglich ist, so dass es in einer rechtwinklich zu seiner Krümmungsebene stehenden Ebene breiter ist.

3. Gasbehandlungsvorrichtung nach Anspruch 2, **dadurch gekennzeichnet, dass** das Gehäuse (1, 101, 201, 301) einen ovalen Querschnitt hat.

4. Gasbehandlungsvorrichtung nach einem der voranstehenden Ansprüche **dadurch gekennzeichnet, dass** die Gasbehandlungselemente HME-Elemente (4, 104) sind.

5. Gasbehandlungsvorrichtung nach Anspruch 4, **dadurch gekennzeichnet, dass** jedes HME-Element eine Spiralrolle eines hygroskopisch behandelten Papiers (4, 104) enthält.

6. Gasbehandlungsvorrichtung nach einem der voranstehenden Ansprüche, **dadurch gekennzeichnet, dass** die Vorrichtung eine gegenüber dem Anschluss (2) angeordnete Saugzugangsöffnung (60) hat.
